# EUROPEAN PATENT SPECIFICATION

(11) **EP 2 113 276 B1**
(45) Date of publication and mention of the grant of the patent: **15.08.2012**
(21) Application number: 09168444.9
(22) Date of filing: 21.05.2007
(51) Int. Cl.: A61M 16/08, A61M 16/10

(54) **Medical heat and moisture exchanger (HME)**
Medizinischer Wärme- und Flüssigkeitstauscher
Échangeur thermo-hydrique médical (HME)

(43) Date of publication of application: 04.11.2009
(62) Divisional of application: 07425304.8
(73) Proprietor: Covidien AG, 8212 Neuhausen am Rheinfall (CH)
(72) Inventor: Zucchi, Giuseppe, 41039 San Possidonio (IT); Belluzzi, Camillo, 46030 Sustinente (IT); Gallini, Sarah, 41034 Finale Emilia (IT)
(74) Representative: Jorio, Paolo

(56) References cited:
- EP-A2- 1 208 866
- WO-A1-96/35911
- US-A- 5 320 096
- US-B1- 6 397 842

## Description

### FIELD

The present invention relates to a medical heat and moisture exchanger (HME).

More specifically, the present invention may be used to advantage, though not exclusively, in anaesthesiology and intensive care, to which the following description refers purely by way of example.

### BACKGROUND

As is known, a heat and moisture exchanger (HME) is a device used in anaesthesiology and intensive care on intubated patients undergoing artificial ventilation.

In such conditions, in fact, the patient's upper air passages being bypassed, and so not assisting in heating and moistening the cold, dry ventilation gas, an HME is inserted in the respiratory circuit, between the so-called "catheter mount" and "patient-side Y connector", to retain heat and moisture from the gas exhaled by the patient and heated and moistened by the lower air passages, and release large part of the retained heat and moisture to the air supply inhaled by the patient.

Inserting an HME in the circuit, however, increases the "circuit dead space", on account of both the location and form of the HME, both of which factors assist in mixing the inhaled and exhaled gases.

Dead space is formed on the side of each physiological (i.e. anatomical and alveolar) dead space, and possibly on the artificial respiratory system side, and constitutes a volume of air taking no part in oxygen-carbon dioxide exchange, which only takes place in alveoli perfused with blood. The physiological dead space therefore obviously varies according to various factors, whereas the portion produced by the respiratory circuit is of an artificial nature and affected by different factors.

Circuit dead space is clinically undesirable, by causing stagnation and mixing of the fresh gas supply to the patient and the gas exhaled by the patient; so much so that this volume is taken into account and compensated for in the ventilation setting.

Moreover, in the case of a pediatric or newborn patient, the gas flow rate and volumes involved make the presence of a stagnant volume of ventilation gas a serious issue.

Nevertheless, in prior-art devices, even the presence of an HME normally poses problems.

A filter formed substantially from a pleated sheet is described in documents EP 1 208 866 (BELLMAFIOK), US 6 397 842 (LEE ANDREW JAMES), US 5 320 096 (HANS LAMBERT) and WO96/35911 (ELASTEK INC). Such filter presents the features of the preamble portion of the present Claim 1.

### SUMMARY

It is therefore an object of the present invention to provide a medical HME designed to eliminate the aforementioned drawbacks, and which at the same time is cheap and easy to produce.

According to the present invention, there is provided a medical HME as claimed in the accompanying Claims.

The HME according to the present invention operates on the principle of eliminating (ideally all) the dead space typically associated with its presence in the respiratory circuit.

The HME according to the present invention is particularly suitable for use in anaesthesiology and intensive care, to passively heat and moisten respiratory gas supply to the patient.

More specifically, the present invention proposes to eliminate the dead space typically associated with an HME located in respiratory circuits, downstream (in the ventilator-patient direction) from the Y connector.

The HME according to the invention comprises, inside, a heat and moisture exchange septum (or filter), which is only swept on one side by the exhaled gas to accumulate heat and moisture.

To the incoming, normally cold, dry inhaled gas, the septum (or filter), which is only swept on the other side by the exhaled gas, releases the accumulated heat and moisture to administer adequately heated, moistened gas to the patient.

Operation as described above is achieved by separating the paths of the two (respectively, inhaled and exhaled) gases on the opposite surfaces of the septum (or filter). In fact, where there is no volume traversed in both respiratory flow directions, there is no dead space.

### BRIEF DESCRIPTION OF THE DRAWINGS

A number of non-limiting embodiments of the present invention will be described by way of example with reference to the accompanying drawings, in which:

Figure 1 shows a three-dimensional view of a first exemplary HME ;

Figure 2 shows a first section A-A of the first exemplary HME in Figure 1;

Figure 3 shows a second section B-B of the HME in Figures 1, 2;

Figure 4 shows an enlarged view of a septum (or filter) employed in the HME in Figures 1, 2, 3;

Figure 5 shows a three-dimensional view of a second exemplary HME;

Figure 6 shows a spread-out sheet from which to make an alternative septum (or filter) for an HME as shown in Figures 1, 2, 3, 4, 5;

Figure 7 shows a first three-dimensional view of the Figure 6 sheet partly folded;

Figure 8 shows a second three-dimensional view of the Figure 6 sheet partly folded;

Figure 9 shows a three-dimensional view of a third exemplary HME;

Figure 10 shows a longitudinal section C-C of the HME in Figure 9;

Figure 11 shows a septum (or filter) employed in the HME in Figures 9 and 10;

Figure 12 shows a longitudinal section of the Figure 11 septum (or filter);

Figure 13 shows a longitudinal section of a fourth exemplary HME ;

Figure 14 shows a three-dimensional view of an embodiment of an HME in accordance with the present invention;

Figure 15 shows an exploded view of the Figure 14 exchanger;

Figure 16 shows a sheet from which to make a septum (or filter) for an HME as shown in Figure 14;

Figure 17 shows a three-dimensional view of a fifth exemplary HME;

Figure 18 shows a septum (or filter) employed in the HME in Figure 17;

Figure 19 shows a cross section E-E of the Figure 18 septum (or filter);

Figure 20 shows (with enlarged details) a sheet from which to make a septum (or filter) as shown in Figures 17, 18, 19.

### DETAILED DESCRIPTION

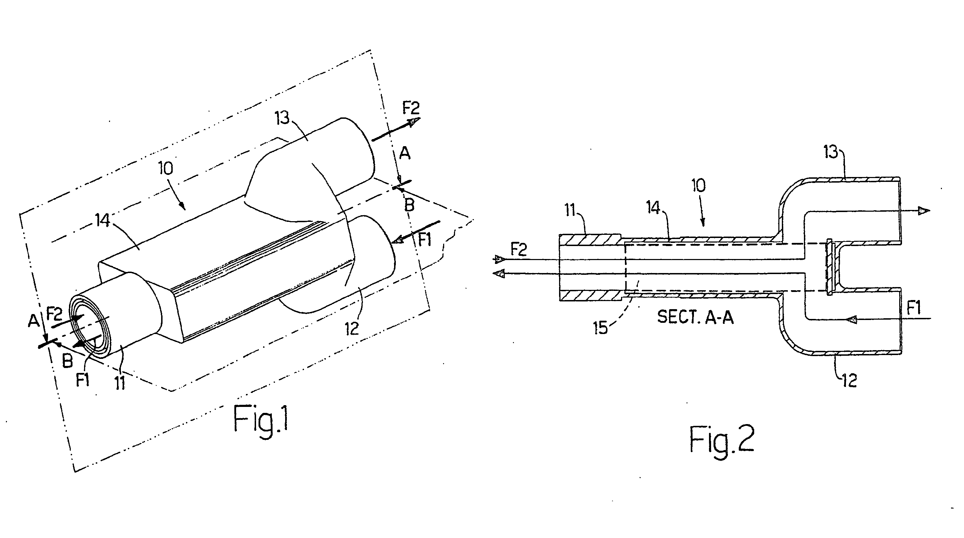

Figures 1, 2, 3, 4 show a first exemplary exchanger (HME) 10.

In this first example, exchanger 10 comprises a substantially tubular first connector 11 connected to a tracheal tube (not shown) of a patient (not shown).

Exchanger 10 also houses a substantially tubular second connector 12, along which flows inhaled gas produced by a ventilator (not shown) and indicated by arrow F1.

Exchanger 10 also comprises a substantially tubular third connector 13, along which exhaled gas F2 is fed back to the ventilator.

Finally, exchanger 10 comprises a substantially parallelepiped-shaped main body 14 interposed between first connector 11, on one side, and connectors 12, 13, on the other.

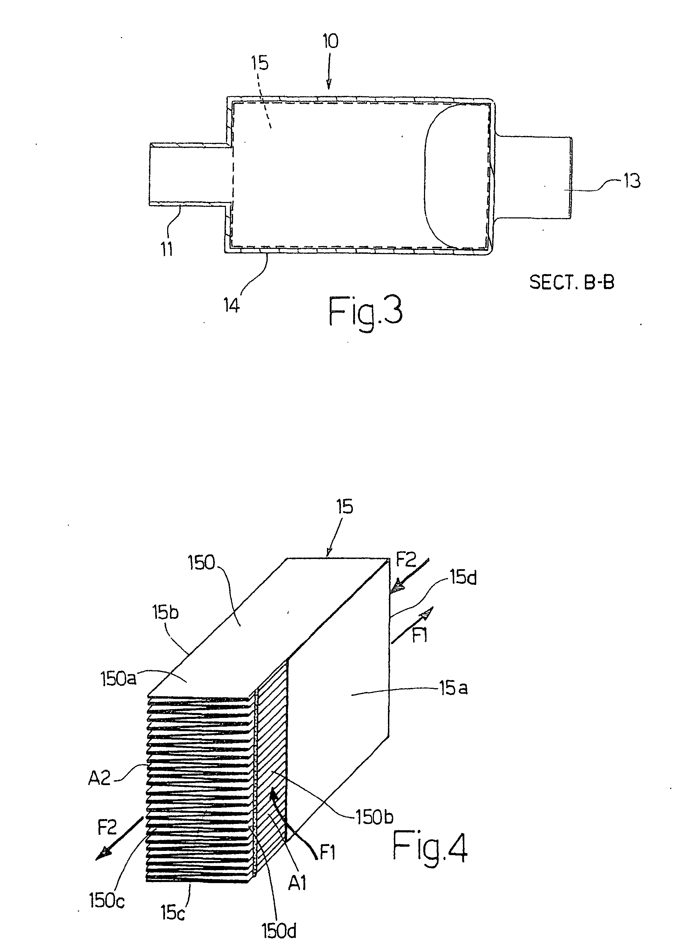

Main body 14 houses a septum (or filter) 15, by which heat and moisture is exchanged between the outflow gas and inflow gas. As shown in detail in Figure 4, septum (or filter) 15 is in the form of a pack, and is formed by pleating a sheet 150 of material capable of retaining and releasing heat and moisture (e.g. hygroscopic paper).

The substantially parallelepiped-shaped pack so formed is potted (sealed) on part of its lateral walls 15a, 15b, and on the whole of a first end wall 15c (Figure 4). For the sake of clarity, the potting coat on first end wall 15c is not shown in Figure 4.

The inhaled gas from connector 12 is thus forced to flow through an exposed area A1 of lateral wall 15a and through a second end wall 15d, opposite first end wall 15c, to connector 11.

Alternately, the exhaled gas flows into septum (or filter) 15 through the non-potted second end wall 15d, is forced to flow through an exposed area A2 of lateral wall 15b, and is ultimately fed through connector 13 to the ventilator.

In this first example, septum (or filter) 15 obviously constitutes no patient dead space, which, in fact, in exchanger 10, is defined solely by the volume of connector 11.

As shown in detail in Figure 4, septum (or filter) 15 is formed by pleating sheet 150 to form a number of identical units 150a.

"Fan-folding" units 150a one on top of the other produces a first set of passages 150b (each defined by two contiguous units 150a connected by a fold 150d) (Figure 4) through which only the inhaled gas (arrow F1) flows; and a second set of passages 150c (each defined by two contiguous units 150a connected and hinged to each other by a fold 150d) (Figure 4) through which only the exhaled gas (F2) flows.

The gases are directed in known manner by two one-way valves (not shown) in the ventilator (not shown). When inhaling, the inhale valve is open and the exhale valve closed; conversely, when exhaling, the exhale valve is open and the inhale valve closed, so that the gases flow along whichever way is clear.

The two gases - inhaled (arrow F1) and exhaled (arrow F2) - therefore never come into direct contact with each other, and simply exchange heat and moisture via septum (or filter) 15, which, as stated, may advantageously, though not necessarily, be made from a sheet 150 of hygroscopic paper.

Exchanger 10 could also be made into an HME filter with practically no dead space, by applying a filtering membrane (not shown) inside connectors 12 and 13 or possibly over exposed areas A1 and A2.

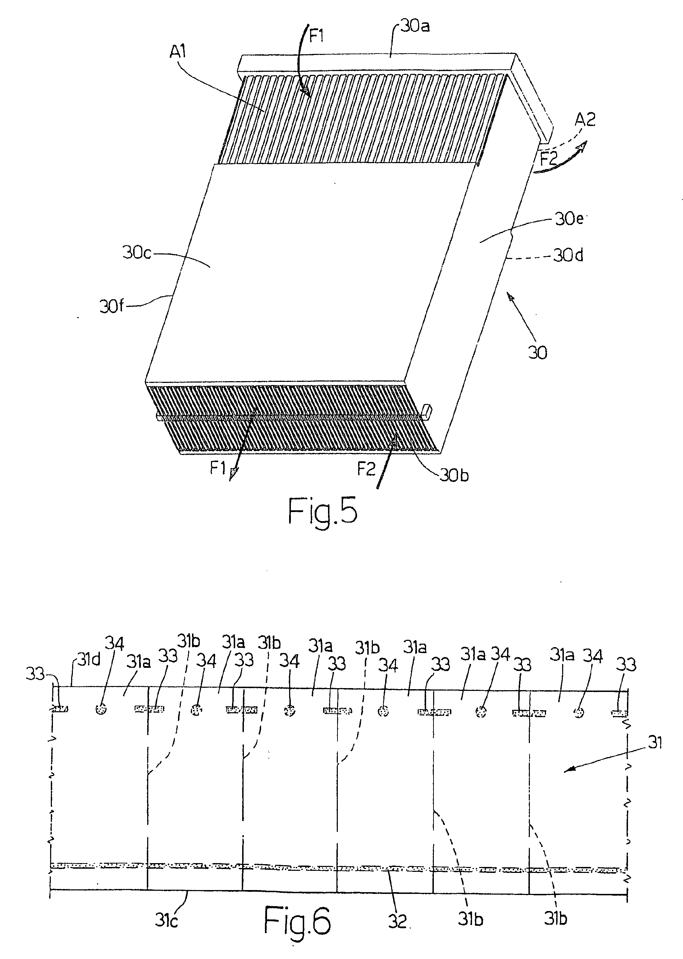

Figures 5, 6, 7, 8 show a second exemplary septum (or filter) 30 for insertion inside main body 14 of exchanger (HME) 10 (in lieu of septum (or filter) 15 described with reference to Figures 1-4).

As shown in Figure 6, septum (or filter) 30 (Figure 5) comprises a substantially rectangular sheet 31 divided into a number of units 31a of equal area. And each two contiguous units 31a are connected by a fold 31b which acts as a hinge.

A continuous streak 32 of potting compound is deposited parallel to and along the whole length of a first long side 31c of sheet 31. Along a second long side 31d (parallel to first side 31c), on the other hand, short segments 33 of potting compound are deposited, each "astride" a respective fold 31b.

As shown in Figure 6, a spot 34 of adhesive and sealing material, e.g. potting compound, is deposited halfway between one fold 31b and another. The same potting compound as for continuous streak 32 is advantageously, though not necessarily, used for both segments 33 and spots 34.

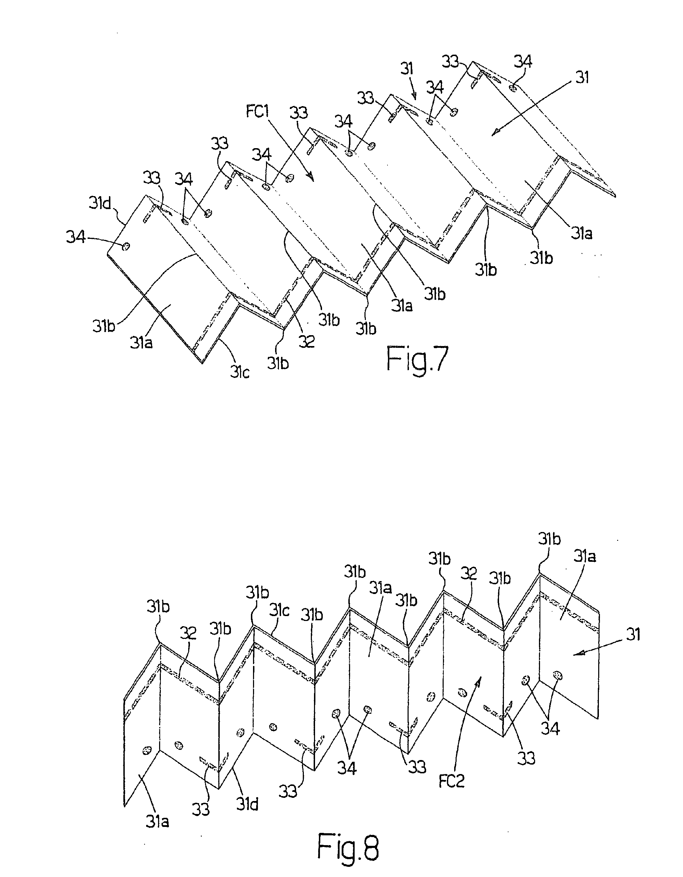

Continuous streak 32, segments 33, and spots 34 are deposited on both a first face FC1 (Figure 7) and a second face FC2 (Figure 8) of continuous sheet 31.

When sheet 31 is pleated at folds 31b, folds 31b are sealed completely along side 31c, coated with continuous streak 32 of potting compound, to form a first end wall 30a (Figure 5); whereas, along side 31d, only the tips of folds 31b are sealed, and the mid-portion of each unit 31a (by spots 34), to form a second end wall 30b (Figure 5).

As shown in Figure 5, in this case too, the substantially parallelepiped-shaped septum (or filter) 30 so formed is potted (sealed) partly on its lateral walls 30c and 30d, and on the whole of its dorsal walls 30e, 30f, so that the inhaled gas from connector 12 (Figure 2) is forced to flow through an exposed area A1 of lateral wall 30c, through septum (or filter) 30, and out through end wall 30b to connector 11 (Figure 1).

Alternately, the exhaled gas flows into septum (or filter) 30 through second end wall 30b, is forced to flow through septum (or filter) 30 and an exposed area A2 of lateral wall 30d, and is ultimately fed to the ventilator through connector 13 (Figure 1).

Spots 34 combine to form a support to maintain correct spacing of the folds, so that the gases flow through the open gaps.

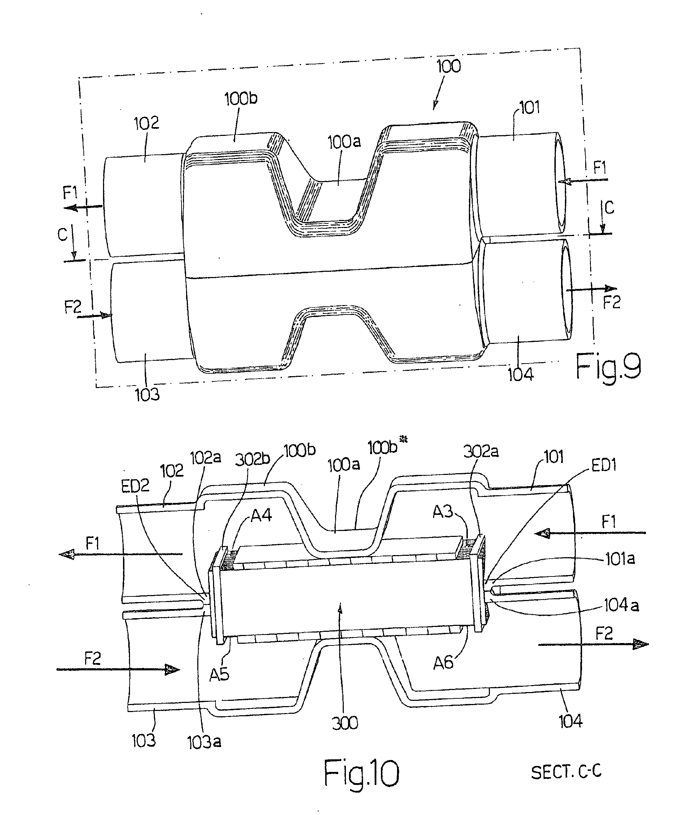

Figures 9, 10, 11, 12 show a third example.

In the third example, an HME 100 (Figure 9) comprises four connectors 101, 102, 103, 104 located downstream from the patient-side Y connector (not shown); and a septum (or filter) 300 (Figure 10) housed in use inside a central portion 100a of HME 100 (Figure 9). In a sense, this third example may be said to be the starting point for the first (Figures 1-4) and second (Figures 5-8) examples.

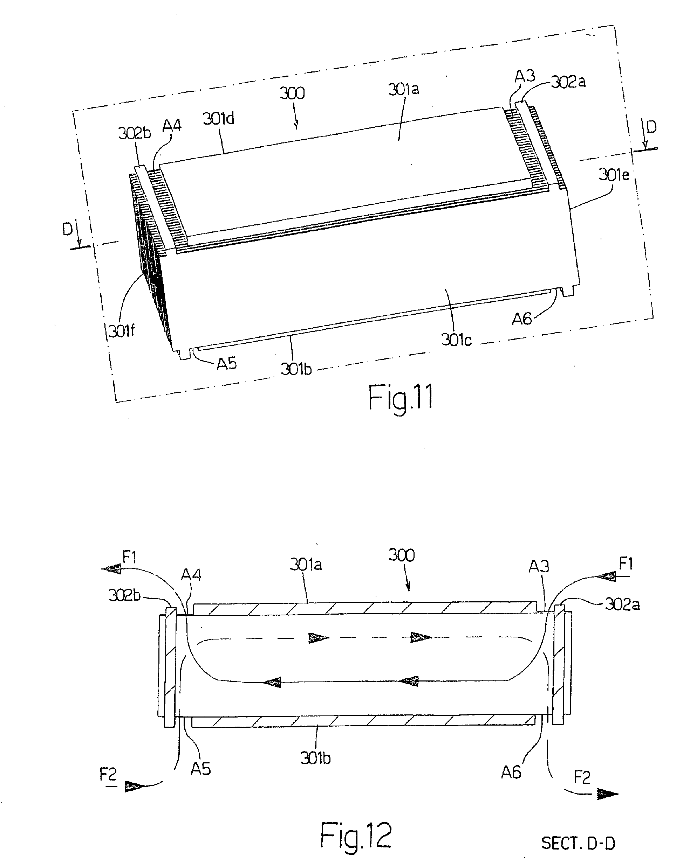

As shown in Figure 11, septum (or filter) 300 comprises two partly potted lateral walls 301a, 301b, and two fully potted dorsal walls 301c, 301d, all of which are potted using the same potting compound. As shown in Figure 12, the inhaled gas (F1) from the ventilator flows in through an exposed area A3 and out to the patient through an exposed area A4, and, as it flows through septum (or filter) 300, is heated and moistened by the gas (F2) exhaled in the preceding cycle (see below).

Similarly, the exhaled gas (F2) flows into septum (or filter) 300 through an exposed area A5 and out through an exposed area A6 to the ventilator. As it flows through septum (or filter) 300, the exhaled gas obviously releases heat and moisture to the walls of a pleated sheet of the type illustrated in the previous examples.

A casing 100b of HME 100, as stated, comprises four connectors 101, 102, 103, 104 - two for inhaled gas (101, 102) and two for exhaled gas (103, 104) - so that the two gas flows indicated by arrows F1, F2 in Figures 10 and 12 are separated. Casing 100b also comprises a depressed central section 100b* forming central portion 100a, so that septum (or filter) 300 is gripped between the walls of casing 100b to prevent gas (inhaled and exhaled) from flowing between the inner wall of central section 100b* and the outer wall of septum (or filter) 300. As a result, the gases (inhaled and exhaled) are forced to only flow through the desired exposed areas A3, A4 and A5, A6 respectively.

In actual use, the inhaled gas from the ventilator flows into septum (or filter) 300 through connector 101, and, by virtue of the potting compound on the central walls 301a, 301b, 301c, 301d, is forced to flow through area A3. Each end of septum (or filter) 300 is fitted with a respective binder 302a, 302b. After sweeping the inside of the passages between the pleats in the sheet, and gathering heat and moisture released to septum (or filter) 300 by the preceding exhale cycle, the inhaled gas flows out through area A4 and through connector 102 to the patient (not shown).

The gas exhaled by the patient flows through connector 103, through area A5 into the passages between the exhale pleats, releases heat and moisture for the next inhale cycle, and flows through area A6 into connector 104, and from there to the ventilator.

In this third example, an important part is played by a first partition ED1, defined on one side by a wall 101a (of connector 101) and a wall 104a (of connector 104); and by a second partition ED2, defined on the other side by a wall 102a (of connector 102) and a wall 103a (of connector 103). Partitions ED1, ED2 rest against end walls 301e, 301f respectively, and provide for separating the inhaled gas (F1) from the exhaled gas (F2).

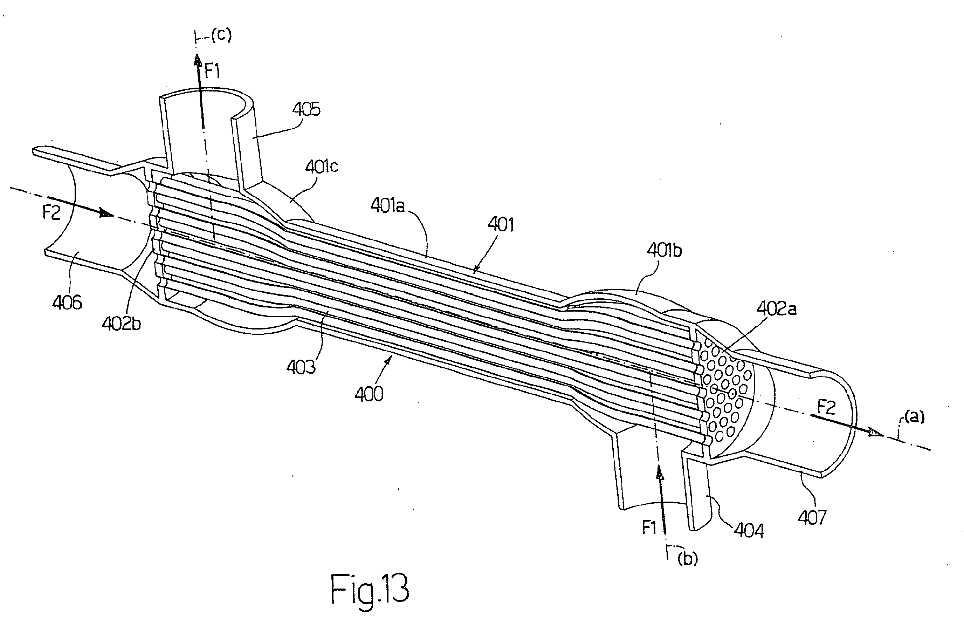

Figure 13 shows a fourth example , which represents a different model from the ones described above, to channel and separate the two gas flows and so reduce dead space, and which basically is constructed in the same way as capillary filters used in haemodialysis (ion exchange between blood and dialysis liquid) and in cardiac surgery to oxygenate blood.

In this example, the gas flows are separated using a number of capillary tubes with given heat and moisture retaining and release properties for a first gas flow; directing a second gas flow outside the capillary tubes; and accordingly locating the connectors to direct the gas flows. Number 400 in Figure 13 indicates a fourth exemplary HME, which comprises a substantially tubular body 401, in turn comprising a substantially tubular central portion 401a, and two end portions 401b, 401c swollen with respect to central portion 401a. Body 401 houses two perforated plates 402a, 402b, each housed in a respective end portion 401b, 401c, and which are substantially perpendicular to an axis (a) of substantial longitudinal symmetry of body 401, and are perforated to support a number of haemodialysis-type capillary tubes 403.

In this fourth example, the inhaled gas (arrow F1) from the ventilator flows through a connector 404 into end portion 401b, along central portion 401a into end portion 401c, and out to the patient through a connector 405. Each connector 404, 405 has a respective axis (b), (c) substantially perpendicular to axis (a). As it flows through, the inhaled gas (F1) flows inside the intercapillary space and sweeps capillary tubes 403, from which it absorbs heat and moisture released by the exhaled gas in the previous cycle (see below).

The gas (F2) exhaled by the patient, in turn, flows through a connector 406 and along the inside of capillary tubes 403, releases heat and moisture to the walls of capillary tubes 403 for the inhaled gas at the next inhale cycle, and ultimately flows out through a connector 407 to the ventilator (not shown).

In the Figure 13 solution too, mixing of the two inhaled and exhaled gas flows is prevented by plates 402a, 402b, which are perfectly airtight. Inside central portion 401a, capillary tubes 403 are preferably closer together to reduce the area of the intercapillary space.

This provision aids in achieving laminar flow of the inhaled gas and, therefore, uniform heat and moisture exchange between the inhaled gas and the gas exhaled in the previous cycle.

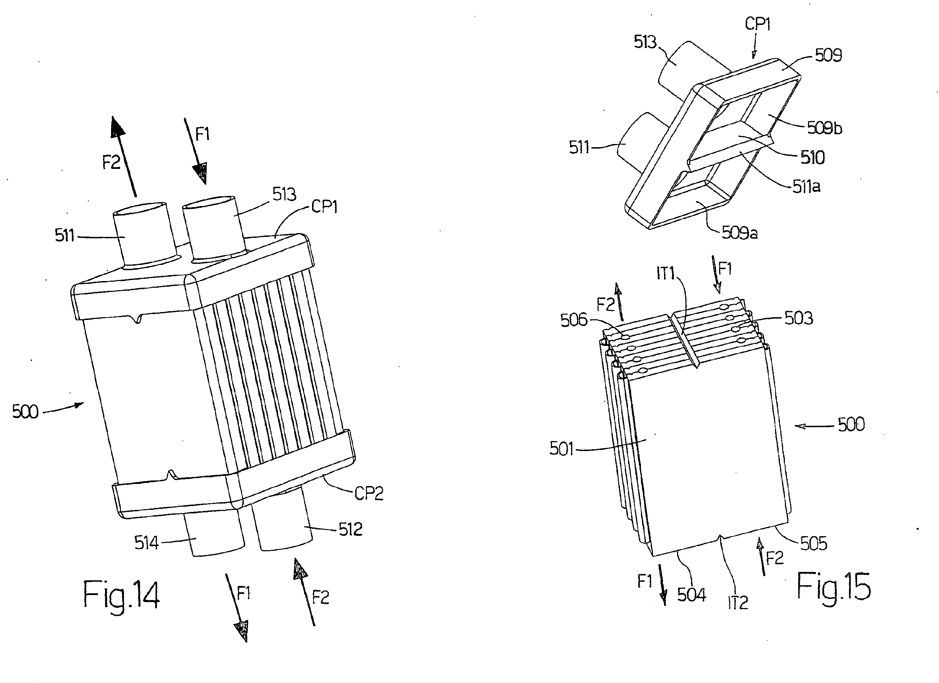

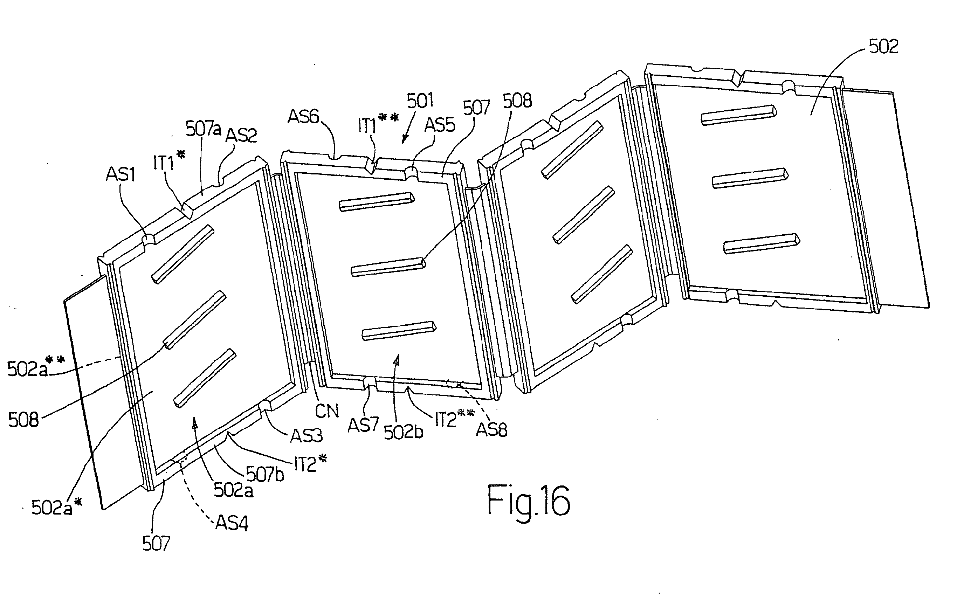

Figures 14, 15, 16 show an embodiment of an HME 500, which employs a filter 501 substantially formed from a sheet 502 (Figure 16) of paper (or film) stamped to form a sort of plate-type exchanger (see below).

Once stamped and pleated, sheet 502 (Figure 16) forms two sets of inhale openings 503, 504 (arrow F1), and two sets of exhale openings 505, 506 (arrow F2) (Figure 15).

As regards the inhaled gas (F1), openings 503 are offset with respect to openings 504; and similarly, as regards the exhaled gas (F2), openings 505 are offset with respect to openings 506, to make the best use of the available heat and moisture exchange surfaces.

With reference to two adjacent, substantially rectangular units 502a, 502b of equal area, a respective rectangular sealing frame 507 is stamped on each unit 502a, 502b; and, as shown in detail in Figure 16, a short side 507a of one unit, e.g. unit 502a, comprises, from left to right, a first half-opening AS1 facing a first face 502a* of the unit; a half-notch IT1* (for reasons explained in detail below); and a second half-opening AS2 facing a second face 502a**, opposite first face 502a*, of the unit.

Similarly, a short side 507b of rectangular unit 502a comprises, from right to left, a third half-opening AS3 facing first face 502a*; a half-notch IT2* (for reasons explained in detail below); and a fourth half-opening AS4 (shown by a dash line in Figure 16) facing second face 502a** opposite first face 502a*. The same also applies to rectangular unit 502b adjacent to rectangular unit 502a. Short side 507a of frame 507 of rectangular unit 502b (like unit 502a) comprises (from right to left) a fifth half-opening AS5 facing first face 502a*; a half-notch IT1**; and a sixth half-opening AS6 facing second face 502a**.

Short side 507b of frame 507 of rectangular unit 502b comprises (from left to right) a seventh half-opening AS7; a half-notch IT2**; and an eighth half-opening AS8 (shown by a dash line in Figure 16). As shown in Figure 16, each rectangular unit 502a, 502b has respective "herringbone" ribs 508, which, besides diverting flow to sweep a larger surface area, also act as spacers between adjacent units 502a, 502b folded one over the other in use. As opposed to being specular, ribs 508 are advantageously equioriented, so that, in use, when sheet 502 is pleated, ribs 508 are superimposed and arranged crosswise to one another to improve gas circulation and make full use of the available heat and moisture exchange surface.

Moreover, half-openings AS1, AS2, AS3, AS4, AS5, AS6, AS7, AS8 are advantageously offset with respect to the relative side of frame 507 to correctly divert the gas flows (inhaled and exhaled). When unit 502b is folded over unit 502a, half-opening AS1 mates with half-opening AS5 to form one of openings 506, and half-opening AS3 mates with half-opening AS7 to form one of openings 505, so that openings 505 are offset with respect to openings 506; and half-notch IT1* mates with half-notch IT1** to form a complete notch IT1, as shown in Figure 15.

As shown in Figure 16, units 502a, 502b are hinged to each other by a hinge CN comprising the fold line and having no stamped material, so as to assist folding of sheet 502 of paper (or film) during the pleating operation. The two specular units 502a, 502b are repeated along the whole length of the paper (or film).

With reference, for example, to unit 502a, second face 502a** is also stamped, but specularly with respect to first face 502a*, so that the result is the same as in Figures 1 and 2, i.e. the inhaled gas (arrow F1) flows through a first number of passages, and the exhaled gas (arrow F2) through a second number of passages. In this case, no potting is required, by virtue of stamped frames 507, which provide for both directing and sealing and separating the two gas flows. Moreover, notches IT1, IT2 provide for attaching a top cover CP1 and a bottom cover CP2 respectively. Top cover CP1 comprises a substantially parallelepiped-shaped main body 509 open on one face.

Inside, main body 509 comprises a tapered partition 510, the free end of which, in use, fits inside notch IT1. Partition 510 divides the space inside main body 509 into two identical portions 509a and 509b. A tubular connector 511 is integral with portion 509a, and a tubular connector 513 is integral with portion 509b (Figure 15). The same also applies to bottom cover CP2, which has tubular connectors 512 and 514 (Figure 14).

To sum up, the inhaled gas (F1) from the ventilator flows through connector 513 to HME 500, and into corresponding openings 503.

As they flow through the system, both gas flows (inhaled and exhaled) are diverted by ribs 508 to effectively sweep both face 502a* and face 502a**, through which heat and moisture exchange takes place. The same obviously also applies to unit 502b and all the other units forming part of filter 501. The incoming inhaled gas (F1) from connector 513 is directed to connector 514 located close to the corner opposite the inflow corner. Flow inside filter 501 takes place with no gas leakage, by virtue of sealing frames 507.

Similarly, the exhaled gas (F2) flows through connector 512 into exhale openings 505, and, as it flows through the system and is diverted by ribs 508, releases heat and moisture as it flows to connector 511 located close to the corner opposite the inflow corner, again with no leakage by virtue of sealing frames 507.

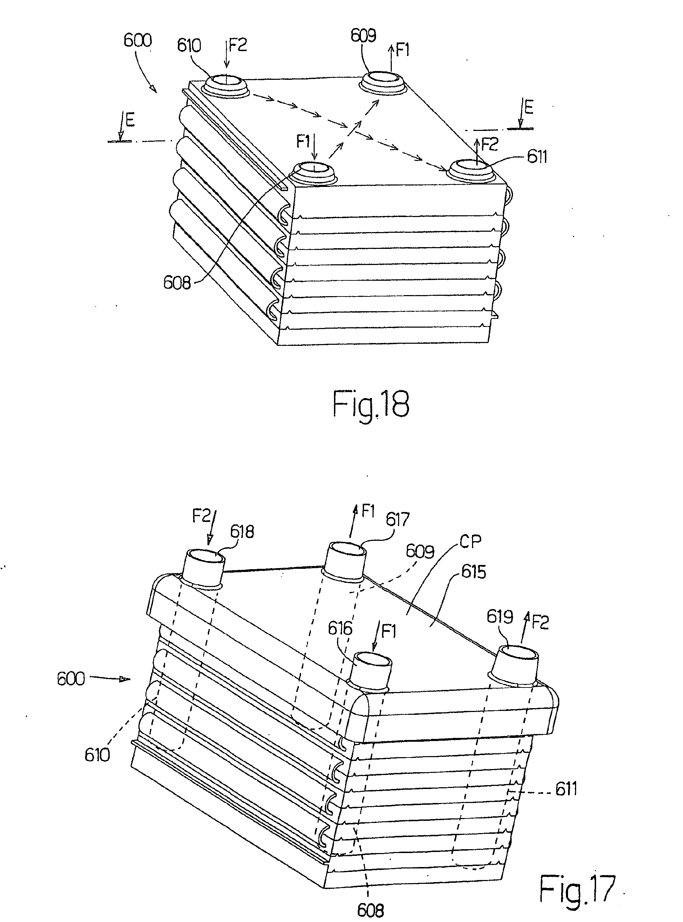

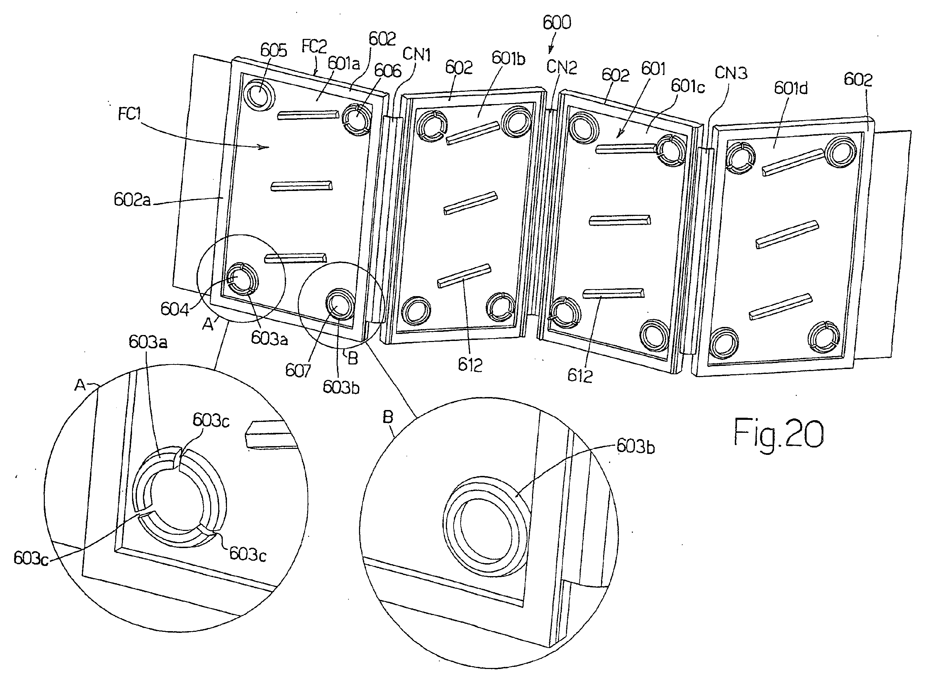

Figures 17, 18, 19, 20 show a fifth example, which relates to a system conceptually similar to the first example (Figures 1-4), second example (Figures 5-8), fourth example (Figure 13), and the embodiment (Figures 14-16), and which also employs a stamped material, such as paper or film, capable of accumulating and releasing heat and moisture. A septum (or filter) 600 comprises a sheet 601 (Figure 20), in turn comprising a number of substantially rectangular units 601a, 601b, 601c, 601d connected in twos by hinges CN1, CN2, CN3.

A sealing frame 602 is stamped on each unit 601a, 601b, 601c, 601d, and, inside frame 602, sealing rings 603 are formed (stamped) about four through holes 604, 605, 606, 607 formed at the four corners of each unit 601a, 601b, 601c, 601d. Of the four sealing rings 603, two rings 603a, located at opposite corners, are characterized by a number of radial openings 603c (Figure 20 - enlarged detail A), so that gas flows through a corresponding through hole 604, 605, 606, 607, as well as through openings 603c, which direct flow on the paper/film to sweep the inner surface of units 601a, 601b, 601c, 601d.

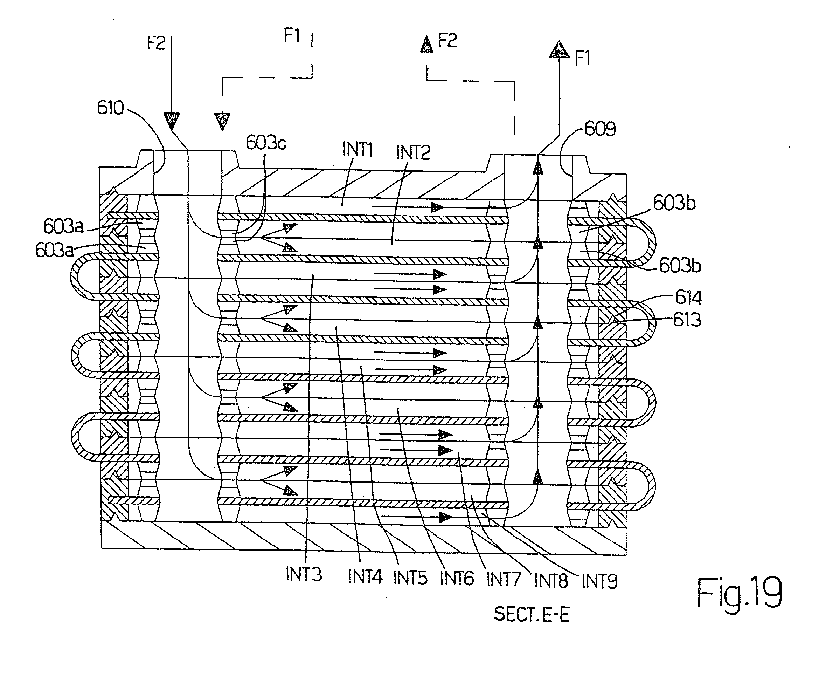

The other two sealing rings 603b, on the other hand, are solid, so that gas only flows through the corresponding through hole 604, 605, 606, 607, and not into the corresponding gaps INT between units 601a, 601b, 601c, 601d. Figure 19 shows clearly how units 601a, 601b, 601c, 601d are stacked, with sealing rings 603a alternating with sealing rings 603b. In other words, sheet 601 (of paper/film) is stamped on both faces, and a front face FC1 is specular with respect to a rear face FC2. Through holes 604, 605, 606, 608 formed in units 601a, 601b, 601c, 601d form four channels 608, 609, 610, 611, as shown in Figures 18 and 19 (only channels 609 and 610 are shown in Figure 19). As a result, the inhaled gas flows inside gaps INT1, INT3, INT5, INT7, INT9, and the exhaled gas inside gaps INT2, INT4, INT6, INT8 (Figure 19).

As before, respective "herringbone" ribs 612 may be provided, which, besides diverting flow to sweep a larger surface area, also act as spacers between adjacent units 601a, 601b, 601c, 601d folded, in use, one on top of the other.

As opposed to being specular, ribs 612 are advantageously, though not necessarily, equioriented, so that, in use, when sheet 601 is pleated, ribs 612 are superimposed and arranged crosswise to one another to improve gas circulation and make full use of the available heat and moisture exchange surface.

Further ribs 613 may be provided on, and extending the whole length of, the long sides 602a of frame 602. When the sheet is pleated, each rib 613 engages a corresponding seat 614 on the next unit 601a, 601b, 601c, 601d to position units 601a, 601b, 601c, 601d correctly when assembling septum (or filter) 600.

Septum (or filter) 600 is also fitted with a cover CP (Figure 17) comprising a substantially parallelepiped-shaped body 615 matching the perimeter of units 601a, 601b, 601c, 601d; and four connectors 616, 617, 618, 619 corresponding with respective channels 608, 609, 610, 611.

To sum up, the inhaled gas (F1) from the ventilator flows through connector 616 into septum (or filter) 600, along channel 608, through gaps INT1, INT3, INT5, INT7, INT9, and out through channel 609 and corresponding connector 617.

Inside gaps INT1, INT3, INT5, INT7, INT9, the inhaled gas (F1) is enriched with heat and moisture accumulated in the previous exhale cycle and made available by virtue of the characteristics of septum (or filter) 600.

Similarly, the exhaled gas (F2) flows through connector 618 into septum (or filter) 600, along channel 610 into gaps INT2, INT2, INT6, INT8, releases heat and moisture to sheet 601 as it flows through, and flows back to the ventilator through channel 611 and corresponding connector 619.

The main advantage of the present invention lies in reducing the dead space inside the ventilation circuit, by reducing the dead space of the HME. Dead space is clinically undesirable, by causing stagnation and mixing of the gases inhaled and exhaled by the patient; so much so that this volume is taken into account and compensated for in the ventilation setting. The advantage afforded by the invention also increases proportionally in the case of a newborn patient, in which the ventilation gas flow rate and volumes involved make the presence of a stagnant volume of ventilation gas a serious issue.

## Claims

1. A filter (501) for an HME comprising a number of substantially rectangular units (502a, 502b) of equal area; the filter **characterized in that** each unit (502a, 502b) has a respective stamped rectangular sealing frame (507) comprising half-openings (AS1, AS2, AS3, AS4; AS5, AS6, AS7, AS8) configured such that when a first unit (502b) is folded onto a second unit (502a), a first half-opening (AS1) of the second unit mates with a first half-opening (AS5) of the first unit to form a first opening (506), and a second half-opening (AS3) of the second unit mates with a second half-opening (AS7) of the first unit to form a second opening (505).

2. A filter (501), as claimed in Claim 1, **characterized in that** the openings (504, 505) are offset with respect to the relative frame (507) to divert flow of the inhaled gas (F1) and exhaled gas (F2).

3. A filter (501), as claimed in Claim 1, **characterized in that** said frame (507) of said second unit (502a) comprises a first half-notch (IT1*) at the top, and a second half-notch (IT2*) at the bottom, and said frame (507) of said first unit (502b) comprises a third half-notch (IT1**) at the top, and a fourth half-notch (IT2**) at the bottom; the first half-notch (IT1*) mating with the third half-notch (IT1**) to form a first complete notch (IT1), and the second half-notch (IT2*) mating with the fourth half-notch (IT2**) to form a second complete notch (IT2).

4. A filter (501), as claimed in Claim 1, **characterized in that** each unit (502a) has a first face (502a*) having two half-openings (AS1, AS3) offset with respect to each other; and a second face (502a**) having two half-openings (AS4, AS4) offset with respect to each other; the first face (502a*) being stamped specularly with respect to the second face (502a**).

5. A filter (501), as claimed in any one of Claims 1 to 4, **characterized in that** each unit (502a, 502b) has respective "herringbone" ribs (508), which are nonspecular but equioriented, so that they are superimposed and arranged crosswise to one another in use.

6. An HME (500) **characterized by** comprising a filter (501) as claimed in any one of Claims 1 to 5, a top cover (CP1) and a bottom cover (CP2).

7. An HME (500), as claimed in Claim 6, **characterized in that** each cover (CP1, CP2) comprises a substantially parallelepiped-shaped main body (509) open on one face; said main body (509) comprising, in turn, an inner partition (510) separating the inhaled gas (F1) from the exhaled gas (F2).

8. An HME (500), as claimed in Claim 7, wherein the partition (510) is tapered, so that its free end is housed, in use, inside a respective said complete notch (IT1; IT2).

9. An HME (500) as claimed in Claim 7 or in Claim 8, **characterized in that** said main body (509) is divided by said partition (510) into a first and second portion (509a, 509b); a first tubular connector (511) being integral with the first portion (509a), and a second tubular connector (513) being integral with the second portion (509b).

10. A ventilating system for use in anaesthesiology and intensive care on intubated patients undergoing artificial ventilation, **characterized in that** it comprises at least one HME as claimed in either of the foregoing Claims 6 to 9.

## Patentansprüche

1. Filter (501) für einen Wärme- und Flüssigkeitstauscher, der eine Anzahl von im Wesentlichen rechteckigen Einheiten (502a, 502b) von gleicher Fläche umfasst;
wobei der Filter **dadurch gekennzeichnet ist, dass** jede Einheit (502a, 502h) einen jeweiligen gestanzten rechteckigen Dichtungsrahmen (507) aufweist, der Halböffnungen (AS1, AS2, AS3, AS4; AS5, AS6, AS7, AS8) umfasst, die derart ausgebildet sind, dass, wenn eine erste Einheit (502b) auf eine zweite Einheit (502a) geklappt wird, eine erste Halböffnung (AS1) der zweiten Einheit zu einer ersten Halböffnung (AS5) der ersten Einheit passt, um eine erste Öffnung (506) zu bilden, und eine zweite Halböffnung (AS3) der zweiten Einheit zu einer zweiten Halböffnung (AS7) der ersten Einheit passt, um eine zweite Öffnung (505) zu bilden.

2. Filter (501) nach Anspruch 1, **dadurch gekennzeichnet, dass** die Öffnungen (504, 505) mit Bezug auf den relativen Rahmen (507) versetzt sind, um den Strom des eingeatmeten Gases (F1) und des ausgeatmeten Gas (F2) umzulenken.

3. Filter (501) nach Anspruch 1, **dadurch gekennzeichnet, dass** der Rahmen (507) der zweiten Einheit (502a) eine erste Halbkerbung (IT1*) an der Oberseite und eine zweite Halbkerbung (IT2*) an der Unterseite umfasst und der Rahmen (507) der ersten Einheit (502b) eine dritte Halbkerbung (IT1**) an der Oberseite umfasst und eine vierte Halbkerbung (IT2**) an der Unterseite umfasst; wobei die erste Halbkerbung (IT1*) zu der dritten Halbkerbung (IT1**) passt, um eine erste vollständige Kerbung (IT1) zu bilden, und die zweite Halbkerbung (IT2*) zu der vierten Halbkerbung (IT2**) passt, um eine zweite vollständige Kerbung (IT2) zu bilden.

4. Filter (501) nach Anspruch 1, **dadurch gekennzeichnet, dass** jede Einheit (502a) eine erste Fläche (502a*) mit zwei Halböffnungen (AS1, AS3) hat, die relativ zueinander versetzt sind; und eine zweite Fläche (502a**) mit zwei Halböffnungen (AS4, AS4) hat, die relativ zueinander versetzt sind; wobei die erste Fläche (502a*) mit Bezug auf die zweite Fläche (502a**) spiegelbildlich gestanzt ist.

5. Filter (501) nach einem der Ansprüche 1 bis 4, **dadurch gekennzeichnet, dass** jede Einheit (502a, 502b) jeweilige "Fischgräten"-Rippen (508) umfasst, die nichtspiegelbildlich, sondern in gleicher Richtung ausgerichtet sind, so dass sie übereinander liegen und während des Gebrauchs kreuzweise zueinander angeordnet sind.

6. Wärme- und Flüssigkeitstauscher (500), **dadurch gekennzeichnet, dass** er einen Filter (501) nach einem der Ansprüche 1 bis 5, eine obere Abdeckung (CP1) und eine untere Abdeckung (CP2) umfasst.

7. Wärme- und Flüssigkeitstauscher (500) nach Anspruch 6, **dadurch gekennzeichnet, dass** jede Abdeckung (CP1, CP2) einen im Wesentlichen parallelepipedförmigen Hauptkörper (509) umfasst, der an einer Fläche offen ist; wobei der Hauptkörper (509) seinerseits eine innere Trennwand (510) umfasst, die das eingeatmete Gas (F1) von dem ausgeatmeten Gas (F2) trennt.

8. Wärme- und Flüssigkeitstauscher (500) nach Anspruch 7, wobei die Trennwand (510) verjüngt ist, so dass ihr freies Ende während des Gebrauchs in einer solchen vollständigen Kerbung (IT1; IT2) aufgenommen ist.

9. Wärme- und Flüssigkeitstauscher (500) nach Anspruch 7 oder Anspruch 8, **dadurch gekennzeichnet, dass** der Hauptkörper (509) durch die Trennwand (510) in einen ersten und einen zweiten Abschnitt (509a, 509b) getrennt wird; wobei ein erster röhrenförmiger Verbinder (511) integral mit dem ersten Abschnitt (509a) ausgebildet ist und ein zweiter röhrenförmiger Verbinder (513) integral mit dem zweiten Abschnitt (509b) ausgebildet ist.

10. Beatmungssystem zur Verwendung in der Anästhesiologie und intensivmedizinischen Behandlung intubierter Patienten, die künstlich beatmet werden, **dadurch gekennzeichnet, dass** es mindestens einen Wärme- und Flüssigkeitstauscher nach einem der vorangehenden Ansprüche 6 bis 9 umfasst.

## Revendications

1. Filtre (501) pour un échangeur de chaleur et d'humidité (HME) comprenant un ensemble d'unités sensiblement rectangulaires (502a, 502b) de surface égale;
le filtre étant **caractérisé en ce que** chaque unité (502a, 502b) a un châssis d'étanchéité rectangulaire poinçonné (507) respectif comprenant des demies ouvertures (AS1, AS2, AS3, AS4 ; AS5, AS6, AS7, AS8) configurées de sorte que lorsqu'une première unité (502b) est pliée sur une seconde unité (502a), une première demie ouverture (AS1) de la seconde unité s'accouple avec une première demie ouverture (AS5) de la première unité pour former une première ouverture (506), et une seconde demie ouverture (AS3) de la seconde unité s'accouple avec une seconde demie ouverture (AS7) de la première unité pour former une seconde ouverture (505).

2. Filtre (501), tel que revendiqué dans la revendication 1, **caractérisé en ce que** les ouvertures (504, 505) sont décalées par rapport au châssis (507) relatif pour dévier l'écoulement du gaz inhalé (F1) et du gaz exhalé (F2).

3. Filtre (501), tel que revendiqué dans la revendication 1, **caractérisé en ce que** ledit châssis (507) de ladite seconde unité (502a) comprend une première demie entaille (IT1*) sur le dessus, et une deuxième demie entaille (IT2*) sur le dessous, et ledit châssis (507) de ladite première unité (502b) comprend une troisième demie entaille (IT1**) sur le dessus, et une quatrième demie entaille (IT2**) sur le dessous ; la première demie entaille (IT1*) s'accouplant avec la troisième demie entaille (IT1**) pour former une première entaille complète (IT1), et la deuxième demie entaille (IT2*) s'accouplant avec la quatrième demie entaille (IT2**) pour former une seconde entaille complète (IT2).

4. Filtre (501), tel que revendiqué dans la revendication 1, **caractérisé en ce que** chaque unité (502a) a une première face (502a*) ayant deux demies ouvertures (AS1, AS3) décalées l'une par rapport à l'autre ; et une seconde face (502a**) ayant deux demies ouvertures (AS4, AS4) décalées l'une par rapport à l'autre ; la première face (502a*) étant poinçonnée de manière spéculaire par rapport à la seconde face (502a**).

5. Filtre (501), tel que revendiqué dans l'une quelconque des revendications 1 à 4, **caractérisé en ce que** chaque unité (502a, 502b) a des nervures respectives "en chevrons" (508), qui sont non spéculaires mais orientées dans la même direction, de sorte qu'elles sont superposées et agencées transversalement les unes par rapport aux autres en utilisation.

6. Échangeur de chaleur et d'humidité (HME) (500) **caractérisé en ce qu'**il comprend un filtre (501) tel que revendiqué dans l'une quelconque des revendications 1 à 5, un couvercle supérieur (CP1) et un couvercle inférieur (CP2).

7. HME (500), tel que revendiqué dans la revendication 6, **caractérisé en ce que** chaque couvercle (CP1, CP2) comprend un corps principal de forme sensiblement parallélépipédique (509) ouvert sur une face; ledit corps principal (509) comprenant, à son tour, une séparation intérieure (510) séparant le gaz inhalé (F1) du gaz exhalé (F2).

8. HME (500), tel que revendiqué dans la revendication 7, dans lequel la séparation (510) est amincie, de sorte que son extrémité libre est reçue, en utilisation, à l'intérieur d'une dite entaille complète (IT1 ; IT2) respective.

9. HME (500) tel que revendiqué dans la revendication 7 ou dans la revendication 8, **caractérisé en ce que** ledit corps principal (509) est divisé par ladite séparation (510) en une première partie et une seconde partie (509a, 509b) ; un premier raccord tubulaire (511) étant d'une seule pièce avec la première partie (509a), et un second raccord tubulaire (513) étant d'une seule pièce avec la seconde partie (509b).

10. Système de ventilation pour une utilisation en anesthésiologie et en soins intensifs sur des patients intubés subissant une ventilation artificielle, **caractérisé en ce qu'**il comprend au moins un HME tel que revendiqué dans l'une ou l'autre des revendications 6 à 9 précédentes.
